(19) Europäisches Patentamt
European Patent Office
Office européen des brevets

(11) Publication number: 0 050 846
A1

(12) # EUROPEAN PATENT APPLICATION

(21) Application number: 81108749.3

(22) Date of filing: 22.10.81

(51) Int. Cl.³: C 07 D 213/64
C 07 D 405/02
//A01N43/40, (C07D405/02, 317/64, 213/64)

(30) Priority: 23.10.80 US 199392

(43) Date of publication of application: 05.05.82 Bulletin 82/18

(84) Designated Contracting States: BE CH DE FR GB IT LI NL SE

(71) Applicant: THE DOW CHEMICAL COMPANY
2030 Abbott Road Post Office Box 1967
Midland, Michigan 48640(US)

(72) Inventor: Au, Andrew Tai Chiu
42 Mackintosh Avenue
Needham Massachusetts 02194(US)

(74) Representative: Weickmann, Heinrich, Dipl.-Ing
Patentanwälte Dipl.Ing.H.Weickmann et al,
Dipl.Phys.Dr.K.Fincke Dipl.Ing.F.A.Weickmann
Dipl.Chem.B.Huber, Dr.-Ing.H.Liska Möhlstrasse 22
D-8000 München 86(DE)

(54) **Preparation of cyano (6-(substituted phenoxy)-2-pyridinyl) methyl esters of 3-(2,2-dihaloethenyl)-2,2-dimethyl cyclopropane carboxylic acids.**

(57) A process for preparing cyano(6-(substituted phenoxy)-2-pyridinyl)methyl esters of 3-(2,2-dihaloethenyl)-2,2-dimethylcyclopropane carboxylic acids wherein an appropriate 6-(substituted phenoxy)picolinaldehyde is reacted with a 3-(2,2-dihaloethenyl)-2,2-dimethylcyclopropane carboxylic acid halide in the presence of an alkali metal cyanide and a solvent.

EP 0 050 846 A1

0050846

# PREPARATION OF CYANO(6-(SUBSTITUTED PHENOXY)-2--PYRIDINYL)METHYL ESTERS OF 3-(2,2-DIHALOETHENYL)--2,2-DIMETHYL CYCLOPROPANE CARBOXYLIC ACIDS

In U.S. Patent 4,163,787, cyano(6-(substituted pyridinyl)methyl esters of 3-(2,2-dihaloethenyl)-2,2--dimethyl cyclopropane carboxylic acids, their preparation by the reaction of an appropriate cyano(6-(substituted phenoxy)-2-pyridine)methanol with a 3-(2,2-dihaloethenyl)--2,2-dimethyl cyclopropane carboxylic acid halide in the presence of a solvent and a hydrogen halide acceptor and their utility as insecticides are taught. The pyridine methanol reactant is prepared by the reaction, at room temperature, of an appropriate 6-(substituted phenoxy)-picolinaldehyde with an excess of an alkali metal cyanide in the presence of an alkali metal bisulfite and water. While the process as taught by U.S. Patent 4,163,787 gives the desired product, other more economical methods of preparation are being sought.

This invention provides a process for preparing cyano(6-(substituted phenoxy)-2-pyridinyl)methyl esters of 3-(2,2-dihaloethenyl)-2,2-dimethyl cyclopropane carboxylic acids corresponding to the formula

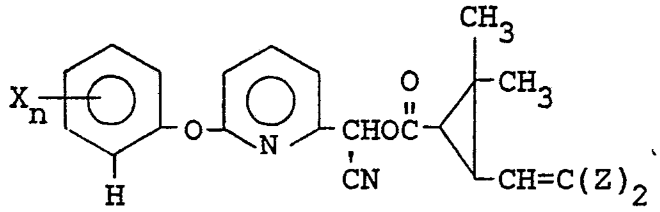

wherein X independently represents alkyl of 1 to 4 carbon atoms, alkoxy of 1 to 4 carbon atoms, alkylthio of 1 to 4 carbon atoms, alkylsulfonyl of 1 to 4 carbon atoms, trifluoromethyl, 3,4-methylenedioxy, chloro, fluoro or bromo; n represents an integer of 0 to 2 and Z represents chloro, fluoro or bromo which comprises reacting an appropriate 6-(substituted phenoxy)picolinaldehyde with a 3-(2,2-dihaloethenyl)-2,2-dimethylcyclopropane carboxylic acid halide (chloride, bromide or fluoride) in substantially equimolar proportions in the presence of an alkali metal cyanide and a solvent, with or without the additional presence of a catalyst.

The reaction scheme can be characterized as follows:

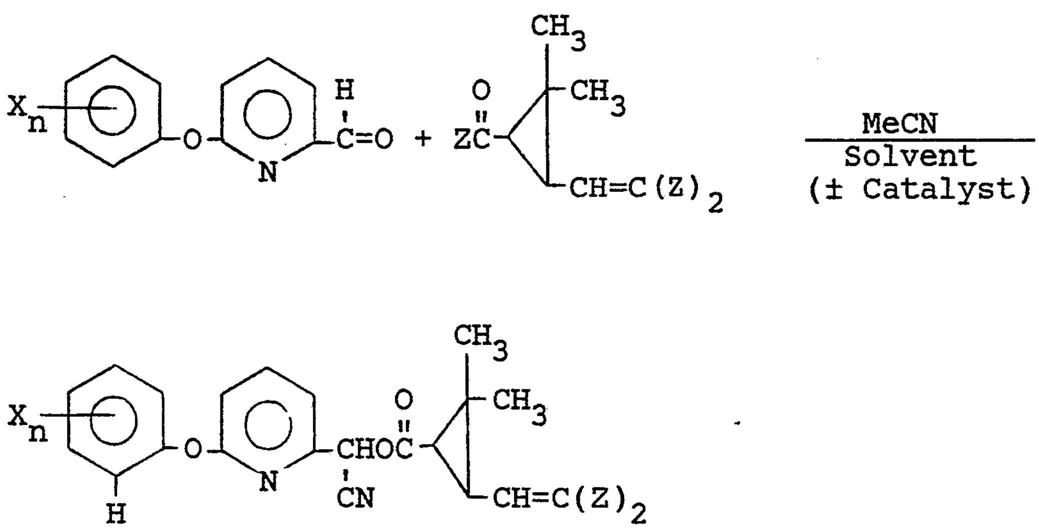

wherein Me is sodium, potassium, lithium or cesium and Z, X and n are as defined. No attempt has been made to present a balanced equation.

In carrying out this process, the aldehyde and acid halide reactants are mixed together in a non-polar solvent such as, for example, carbon tetrachloride, methylene chloride, toluene or chlorform.

This mixture is then admixed with the alkali metal cyanide, as an aqueous solution. Representative cyanides include the cyanides of sodium, potassium, lithium and cesium with sodium cyanide being preferred considering the relative costs of the different cyanides. It has been found that the reaction rate is aided by the use of excess cyanide and it is preferred that from 1.5 to 2.0 molar equivalents of the cyanide be employed although amounts of from 1 to 10 molar equivalents can be used.

The process proceeds well with or without a catalyst. The main advantage in the use of a catalyst is in the slight increase in the reaction rate and in the increase in the selectively to the production of the desired compound.

If it is decided to use a catalyst, essentially any compound from the known class of quaternary ammonium and phosphonium salts can be employed. Suitable quaternary ammonium and phosphonium salts have a minimum solubility of at least one weight percent in the liquid reaction medium at 25°C and normally have a total aggregate carbon content of at least 10 carbon atoms and preferably from

12 to 31 carbon atoms. The ammonium and phosphonium salts can be represented by the formula

$$R_1'R_2'R_3'R_4'Q^{\oplus}A^{\ominus} (X)$$

wherein $R_1'$-$R_4'$ are hydrocarbyl groups (e.g., alkyl, aryl, alkaryl, aralkyl, cycloalkyl, etc.) and $Q^{\oplus}$ is a quaternized atom of nitrogen or phosphorus. Additionally, in (X)$R_1'$ can join with $R_2'$ to form a 5- or 6-membered heterocyclic compound having at least one quaternized nitrogen or phosphorus atom in the ring and may also contain one non-adjacent atom of nitrogen, oxygen or sulfur within the ring. Typically, $R_1'$-$R_4'$ in (X) are hydrocarbyl groups of from 1 to about 12 carbon atoms. $A^{\ominus}$ is an inert neutralizing anion and may be varied to convenience. By "inert" is meant inert in the instant process. Chloride and bromide are the preferred anions but other suitable anions include, for example, fluoride, bisulfate, hydroxide, perchlorate, nitrate and tosylate. The following compounds are illustrative: tetraalkyl ammonium salts, such as tetra-n-butyl-, tetrahexyl-, tri-n-butylmethyl-, cetyltrimethyl-, trioctylmethyl- and tridecylmethyl ammonium chlorides, bromides, bisulfates, tosylates, etc.; aralkylammonium salts, such as tetrabenzylammonium chloride, benzyltrimethyl-, benzyltriethyl-, benzyltributyl-, and phenethyltrimethylammonium chlorides, bromides, etc.; arylammonium salts, such as triphenylmethylammonium fluoride, chloride or bromide N,N,N-trimethylanilinium bromide, N,N-diethyl-N-methylanilinium bisulfate, trimethylnaphthylammonium chloride, p-methylphenyltrimethylammonium chloride or tosylate, etc.; 5- and 6-membered heterocyclic compounds

containing at least one quaternized nitrogen atom in the ring, such as N-methylpyridinium chloride or methyl sulfate, N-hexyl pyridinium iodide, (4-pyridyl)-trimethylammonium chloride, 1-methyl-1-azabicyclo[2.2.1]heptane bromide, N,N-dibutylmorpholinium chloride, N-ethylthiazolium chloride, N-butylpyrrolium chlorides, etc., and the corresponding phosphonium salts and other like compounds.

The ammonium salts are currently preferred over the phosphonium salts due to cost and commercial availability. The most preferred catalysts are benzyltrimethyl-, benzyltriethyl-, tetra-n-butyl and tri-n-butylmethyl ammonium salts.

The quaternary ammonium and phosphonium salts are used in the process in amounts from 0.1 to 20 mole percent, based on the reactants, but amounts of from 0.5 to 10 mole percent are generally preferred.

The reaction can be conducted over a wide temperature range such as, for example, from -30° to 100°C. It is preferred to employ temperatures of from -20°C to room temperature.

The reaction is usually complete in from 2 to 5 hours. However, times of from 1 hour to several days can be employed without overly affecting the formation of the desired product.

The reaction produces as a by-product a tar which must be removed as a part of the purification procedures. The amount of tar produced can be controlled

to some extent by the selection of the solvent and to a lesser extent through the selection of temperature and the use of a catalyst.

At the completion of the reaction, the desired product can be separated by extracting it from the reaction mixture with a solvent such as, for example, light petroleum ether. The product can then be separated from the ether by conventional separatory techniques such as decantation, distillation and evaporation.

Alternatively, the tar can be conveniently removed by passing the reaction mixture through a fixed bed of an adsorbent such as, for example, silica gel, alumina or charcoal.

<u>Example 1</u> - Preparation of 3-(2,2-dichloroethenyl)-2,2--dimethylcyclopropane carboxylic acid:cyano-(6-phenoxy-2-pyridinyl)methyl ester

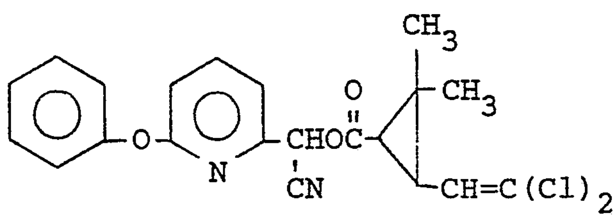

A solution was prepared comprising 19 grams of 6-(phenoxy)picolinaldehyde and 23.7 grams of 3-(2,2--dichloroethenyl)-2,2-dimethylcyclopropane carboxylic acid chloride in 200 milliliters of carbon tetrachloride containing 0.57 grams (2 mole percent) of tetra-*n*-butylammonium chloride. To this solution, stirring at -10°C, was slowly added an aqueous solution of 10 grams of sodium

cyanide in 60 milliliters of water. After 2.5 hours of stirring, the reaction was substantially completed as shown by gas-liquid chlormatography. The organic layer which formed upon standing was separated, washed with water, washed with a saturated sodium bicarbonate solution, dried over magnesium sulfate and concentrated to yield a yellow oil. A 10 gram sample of this oil was further purified by being extracted with light petroleum ether. The desired product was obtained in a yield of 9.75 grams.

<u>Example 2</u> - Preparation of 3-(2,2-dichloroethenyl)-2,2-dimethylcyclopropane carboxylic acid:cyano-(6-phenoxy-2-pyridinyl)methyl ester

To a mixture of 5.0 grams (0.1 mole) of sodium cyanide in 25 milliliters of water at room temperature was added over a 15 minute period, with stirring, 50 milliliters of toluene containing 10.3 grams (0.052 mole) of 6-(phenoxy)picolinaldehyde and 11.35 grams (0.05 mole) of 3-(2,2-dichloroethenyl)-2,2-dimethylcyclopropane carboxylic acid chloride. A mild exotherm developed during the addition and the mixture was cooled to room temperature on a water bath. The mixture was stirred for four hours at room temperature. The organic phase was separated, washed with a saturated sodium bisulfite solution, then with a sodium bicarbonate solution. The reaction mixture was then dried and then passed through a sinter glass funnel containing 65 grams of silica gel. The filtrate was concentrated to yield 21.0 grams of a pale yellow viscous oil.

In both of the above examples, the structure of the compound was confirmed by both the infrared and nuclear magnetic resonance spectra thereof. It was further noted that the purity of the product was at least 90 percent.

1. A process for preparing cyano(6--(substituted phenoxy)-2-pyridinyl)methyl esters of 3-(2,2-dihaloethenyl)-2,2-dimethylcyclopropane carboxylic acids corresponding to the formula

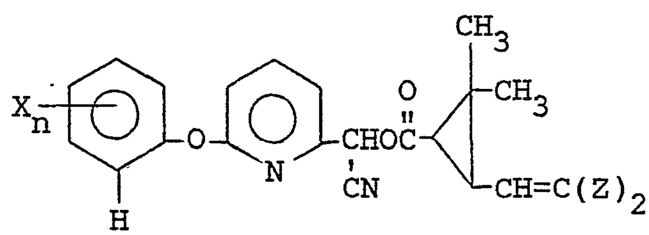

wherein X independently represents alkyl of 1 to 4 carbon atoms, alkoxy of 1 to 4 carbon atoms, alkylthio of 1 to 4 carbon atoms, alkylsulfonyl of 1 to 4 carbon atoms, trifluoromethyl, 3,4-methylenedioxy, chloro, fluoro or bromo; n represents an integer of 0 to 2 and Z represents chloro, fluoro or bromo characterized by reacting a 6-(substituted phenoxy)picolinaldehyde with a 3-(2,2-dihaloethenyl)-2,2-dimethylcyclopropane carboxylic acid halide in the presence of an alkali metal cyanide and a solvent.

2. Process of Claim 1 characterized in that the 6-(substitutedphenoxy)picolinaldehyde and the 3-(2,2--dihaloethenyl)-2,2-dimethylcyclopropanol carboxylic acid halide reactants are employed in substantially equimolar proportions.

3. Process of Claim 2 characterized in that the 3-(2,2-dihaloethenyl)-2,2-dimethyl cyclopropane carboxylic acid halide reactant is the chloride.

4. Process of Claim 3 characterized in that the alkali metal cyanide reactant is sodium cyanide.

5. Process of Claim 4 characterized in that from 1.5 to 2.0 molar equivalents of the sodium cyanide is employed.

6. Process of Claim 5 characterized in that the reaction is conducted at a temperature in the range of from -30° to 100°C.

7. Process of Claim 6 characterized in that the reaction is conducted at a temperature in the range of from -20°C to room temperature.

8. Process of Claim 7 characterized in that 6-(phenoxy)picolinaldehyde is reacted with 3-(2,2-dichloroethenyl)-2,2-dimethylcyclopropane carboxylic acid chloride and the product is 3-(2,2-dichloroethenyl)-2,2--dimethylcyclopropane carboxylic acid:cyano-(6-phenoxy--2-pyridinyl)methyl ester.

European Patent Office

# EUROPEAN SEARCH REPORT

0050846

Application number

EP 81108749.3

| Category | Citation of document with indication, where appropriate, of relevant passages | Relevant to claim | CLASSIFICATION OF THE APPLICATION (Int. Cl.³) |
|---|---|---|---|
| | GB - A - 2 038 817 (NISSAN CHEMICAL) (30-07-1980)<br>+ Abstract; preparations 4-6; claim 9 +<br>-- | 1-5 | C 07 D 213/64<br>C 07 D 405/02//<br>A 01 N 43/40<br>(C 07 D 405/02<br>C 07 D 317/64<br>C 07 D 213/64) |
| A | GB - A - 2 037 282 (SUMITOMO) (09-07-1980)<br>+ Page 4, lines 35-65; examples 6,7; claims 7,8 +<br>-- | 1-5 | |
| D | US - A - 4 163 787 (MALHOTRA)<br>+ Abstract +<br>---- | 1 | |

TECHNICAL FIELDS SEARCHED (Int. Cl.³)

C 07 D 213/00
C 07 D 405/00
C 07 C 121/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 07-01-1982 | HOCHHAUSER |

EPO Form 1503.1 06.78